## Europäisches Patentamt
## European Patent Office
## Office européen des brevets

(19)

(11) Publication number: **0 015 616**
**B1**

(12) **EUROPEAN PATENT SPECIFICATION**

(45) Date of publication of patent specification: **30.06.82**

(21) Application number: **80200174.3**

(22) Date of filing: **29.02.80**

(51) Int. Cl.³: **C 07 C 47/54,**
**C 07 C 45/78, C 07 C 45/82**

(54) Method for the purification of benzaldehyde.

(30) Priority: **02.03.79 NL 7901670**

(43) Date of publication of application:
**17.09.80 Bulletin 80/19**

(45) Publication of the grant of the patent:
**30.06.82 Bulletin 82/26**

(84) Designated Contracting States:
**BE DE FR GB IT NL**

(56) References cited:
**FR - A - 2 240 903**

(73) Proprietor: **STAMICARBON B.V.**
**Postbus 10**
**NL-6160 MC Geleen (NL)**

(72) Inventor: **Jongsma, Cornelis**
**Drossaertweide 1**
**NL-6438 HS Oirsbeek (NL)**

(74) Representative: **Hoogstraten, Willem Cornelis**
**Roeland et al,**
**OCTROOIBUREAU DSM Postbus 9**
**NL-6160 MA Geleen (NL)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Method for the purification of benzaldehyde

The invention relates to a method for the purification of benzaldehyde in the presence of water, in particular of benzaldehyde prepared by oxidation of toluene with a gas containing molecular oxygen.

Benzaldehyde is an important starting material in chemical synthesis, for instance in the synthesis of flavours and fragrances. For such applications, the benzaldehyde is often required to have a high degree of purity. However, crude benzaldehyde, in particular benzaldehyde prepared by oxidation of toluene with a gas containing molecular oxygen, contains troublesome impurities, which are very difficult to remove. In particular, it is very difficult to prepare from the crude benzaldehyde a product which meets olfactory specifications. The presence of impurities manifests itself in the rather early discolouration of stored benzaldehyde, occurring even at a very low concentration (ppm by wt. level) of the impurities.

According to the Japanese patent publication No. 24.467/74, it was sought to purify crude benzaldehyde by treating it with water containing sodium hydroxide. This purification method does not give satisfactory results, however. It appears that the treated benzaldehyde still discolours rather soon.

According to the French application 2 240 903 acidic impurities can be removed from an organic liquid being immiscible with water by contacting the organic liquid with a bed of solid alkaline granules. Also this purification method does not give satisfactory results.

The objective of the invention is to provide a solution to said problem. According to the invention, pure benzaldehyde is obtained by treating impure benzaldehyde simultaneously with water and zinc or aluminium. The treated benzaldehyde is subsequently distilled. It is also possible to separate the phases formed in the treatment, and to distil the organic phase only. The zinc or aluminium is normally applied in quantities ranging from 0.1 to 1000 mmole per kg of benzaldehyde, often in quantities ranging from 1 to 200 mmole per kg of benzaldehyde.

A suitable temperature range for the purification of benzaldehyde according to the invention is from the freezing point of water under the reaction conditions (about 270 K) to 500 K. Temperatures higher than 500 K may also be used. Particularly suitable are temperatures between 280 and 400 K. The duration of the treatment may vary between, for instance, 0.1 and 4 hours. The reaction pressure is not critical, though it should be such that the liquid phase is maintained. The pressure may be, for instance, between 10 and 1000 kPa. A pressure approximately equal to the atmospheric pressure, for instance between 50 and 200 kPa, is preferable for practical reasons.

Suitable quantities of water for the purification of benzaldehyde are, for instance, 1—1,000 g per kg of benzaldehyde. Particularly suitable are quantities of 10—150 g per kg of benzaldehyde.

Distillation of the treated benzaldehyde may be carried out at atmospheric or elevated pressure, but preferably at reduced pressure, for instance a pressure of 2—35 kPa.

In the treatment according to the invention, the loss of benzaldehyde is very small and may be in the order of 1—5% by wt. Benzaldehyde meeting olfactory specifications may be obtained even from crude benzaldehyde prepared by oxidation of toluene.

To shorten the required treatment time and/or to lower the required treatment temperature, a base may be added to the water. Suitable bases are water-soluble bases, such as hydroxides and carbonates of alkali and alkaline earth metals, ammonia, or water-soluble organic bases like amines. Particularly suitable are the hydroxides and carbonates of sodium and potassium, and calcium hydroxide. Suitable quantities are up to 5000 mmoles of base per kg of benzaldehyde, in particular up to 500 mmoles per kg of benzaldehyde. The quantity of base is calculated as the equivalent quantity of NaOH. It is highly surprising that crude benzaldehyde can be purified in this manner. For it is known that benzaldehyde is apt to react with a hydroxide solution in a Canizarro reaction, with formation of benzyl alcohol (see for instance Roberts and Caserio, Modern Organic Chemistry, W.A. Benjamin, New York, Amsterdam, 1967, pp. 330, 331, 628). It appears to be possible, though, to purify benzaldehyde with a hydroxide solution and an ignoble metal without a substantial loss of benzaldehyde.

In a suitable mode of realization of the method according to the invention, a mixture of the impure benzaldehyde and water, to which a base may have been added, is passed over a bed containing solid metal particles. Particularly suitable for this purpose is a bed containing zinc dust. There is no need then to remove metal particles from the reaction mixture after the treatment. An additional advantage of this mode of realization is the long period of operation of the equipment due to the very slow depletion of the metal present in the bed.

In another suitable mode of realization of the method according to the invention, which may be combined with the above mentioned mode of realization, the impure benzaldehyde is treated with base-free water and a metal which is less noble than hydrogen, after which the resultant liquid phases are distilled together, without previous separation. The advantage of this mode of realization is that the sometimes difficult separation of the aqueous phase and the organic phase can be omitted. If so desired, it may even be omitted to free the reaction mixture of the metal particles remaining after the treatment. In this mode of realization it is advantageous to use as little water as possible in order to restrict the amount of distillation energy needed.

The invention will be elucidated further by means of the following non-restrictive examples and comparative experiment. The colour value in degrees Hazen (°H) was determined by ASTM D 1209/62.

### Example I

A sample of benzaldehyde, prepared by oxidation of toluene in the liquid phase by means of a gas containing molecular oxygen with the use of a homogeneous cobalt catalyst, was treated with 0.5% by wt. zinc powder and 10% by vol. water for 0.5 hour at 353 K and atmospheric pressure. After removal of the zinc powder, the mixture was distilled in a sieve-tray column with 30 trays at a top pressure of 20 kPa and with a reflux ratio of 1:3. The colour value of the main fraction was below the detection limit of 5°H. This main fraction was divided into two portions. One portion was heated for 6 hours under a nitrogen atmosphere. The colour value rose to 25°C. The other portion was stored for 30 days in a dark bottle under a nitrogen atmosphere. At the end of this period, the colour value has risen to 15°H.

### Example II

A sample of the same liquid crude benzaldehyde as used in example I was treated with 0.9% by wt. zinc powder and 12 vol.% 3 N aqueous sodium hydroxide solution for 0.5 hour at 283 K and atmospheric pressure. After separation of the phases, the organic phase was distilled under the same circumstances as in example I. The colour value of the main fraction was below the detection limit of 5°H. This main fraction was divided into two portions. One portion was heated for 6 hours under a nitrogen atmosphere. The colour value rose to 15°H. The other portion was stored for 30 days in a dark bottle under a nitrogen atmosphere. At the end of this period, the colour value had risen to 10°H.

### Example III

A sample of the same liquid crude benzaldehyde as used in example I was treated with 0.5% by wt. aluminium powder and 50% by vol. 3 N aqueous sodium hydroxide solution for 0.5 hour at 283 K and atmospheric pressure. After separation of the phases, the organic phase was distilled under the same circumstances as in example I. The colour value of the main fraction was below the detection limit of 5°H. This main fraction was divided into two portions. One portion was heated for 6 hours under a nitrogen atmosphere. The colour value rose to 15°H. The other portion was stored for 30 days in a dark bottle under a nitrogen atmosphere. At the end of this period, the colour value had risen to 10°H.

### Comparative experiment

A sample of the same liquid crude benzaldehyde as used in example I was distilled without previous treatment, under the same circumstances as in example I. The colour value of the main fraction was 25°H. This main fraction was divided into two portions. One portion was heated under a nitrogen atmosphere. After 0.2 hour the colour value of this portion had already risen to over 100°H. The other portion was stored for 30 days in a dark bottle under a nitrogen atmosphere. At the end of this period, the colour value had risen to 50°H.

## Claims

1. Method for the preparation of pure benzaldehyde in the presence of water, characterized in that impure benzaldehyde is simultaneously treated with water and zinc or aluminium.

2. Method according to claim 1, characterized in that the impure benzaldehyde was obtained by oxidation of toluene with a gas containing molecular oxygen.

3. Method according to claim 1 or 2, characterized in that the treatment is carried out at a temperature between the freezing point of the water under the reaction conditions, and 500 K.

4. Method according to any one of the claims 1—3, characterized in that a base is added to the water.

5. Method according to claim 4, characterized in that a hydroxide of sodium, potassium and/or calcium or a carbonate of sodium and/or potassium is used.

6. Method according to any one of the claims 1—5, characterized in that a mixture of the impure benzaldehyde in water, which may contain a base, is passed over a bed containing solid particles of zinc or aluminium.

7. Method according to claim 6, characterized in that a bed containing zinc dust is used.

8. Method according to any one of the claims 1—3, 6 and 7, characterized in that the impure benzaldehyde is treated with base-free water and zinc or aluminium after which the resultant liquid phases are distilled together, without previous separation.

## Patentansprüche

1. Verfahren zur Herstellung von reinem Benzaldehyd in Anwesenheit von Wasser, dadurch gekennzeichnet, dass unreines Benzaldhyd gleichzeitig mit Wasser und Zink oder Aluminium behandelt wird.

2. Verfahren nach Anspruch 1, dadurch gekennzeichnet, dass das unreine Benzaldehyd durch Oxydation von Toluol mit einem molekularen Sauerstoff enthaltenden Gas erhalten wurde.

3. Verfahren nach einem der Ansprüche 1 oder 2, dadurch gekennzeichnet, dass die Behandlung bei einer Temperatur durchgeführt wird die zwischen dem Gefrierpunkt des Wassers unter den Reaktionsbedingungen und 500 K liegt.

4. Verfahren nach einem der Ansprüche 1—3, dadurch gekennzeichnet, dass dem Wasser eine Base zugesetzt wird.

5. Verfahren nach Anspruch 4, dadurch gekennzeichnet, dass ein Natrium-, Kalium- und/oder Calciumhydroxid bzw. ein Natrium- und/oder Kalium-carbonat verwendet wird.

6. Verfahren nach einem der Ansprüche 1—5, dadurch gekennzeichnet, dass eine Mischung des unreinen Benzaldehyds in Wasser, das eine Base enthalten darf, über eine Lage von festen Zink- oder Aluminum-teilchen geleitet wird.

7. Verfahren nach Anspruch 6, dadurch gekennzeichnet, dass eine Lage Zinkstaub verwendet wird.

8. Verfahren nach einem der Verfahren 1—3, 6 und 7, dadurch gekennzeichnet, dass durch unreine Benzaldehyd mit basefreiem Wasser und Zink oder Aluminium behandelt wird, wonach die resultierenden flüssigen Phasen zusammen destilliert werden, und zwar ohne vorherige Trennung.

**Revendications**

1. Procédé de préparation de benzaldéhyde pur en présence d'eau, caractérisé en ce que le benzaldéhyde impur est traité simultanément avec de l'eau et du zinc ou de l'aluminium.

2. Procédé selon la revendication 1, caractérisé en ce que le benzaldéhyde impur est obtenu par l'oxydation de toluène à l'aide d'un gaz contenant de l'oxygène moléculaire.

3. Procédé selon l'une des revendications 1—2, caractérisé en ce que le traitement est effectué à une température située entre le point de congélation de l'eau dans les conditions de réaction et 500 K.

4. Procédé selon l'une des revendications 1 à 3, caractérisé en ce qu'on ajoute une base à l'eau.

5. Procédé selon la revendication 4, caractérisé en ce qu'on utilise un hydroxyde de sodium, de potassium et/ou de calcium ou un carbonate de sodium et/ou de potassium.

6. Procédé selon l'une des revendications 1 à 5, caractérisé en ce que le mélange du benzaldéhyde impur dans de l'eau, qui peut contenir une base, est conduit sur un lit contenant des particules solides de zinc ou d'aluminium.

7. Procédé selon la revendication 6, caractérisé en ce qu'on utilise un lit contenant de la poudre de zinc.

8. Procédé selon l'une des revendications 1 à 3, 6 en 7, caractérisé en ce que le benzaldéhyde impur est traité avec de l'eau exempte de base et avec du zinc ou de l'aluminium, après quoi les phases liquides résultantes sont distillées ensemble sans traitement préalable.